# EUROPÄISCHE PATENTANMELDUNG

(11) **EP 3 646 853 A1**
(43) Veröffentlichungstag der Anmeldung: **06.05.2020**
(21) Anmeldenummer: 19204549.0
(22) Anmeldetag: 22.10.2019
(51) Int. Cl.: A61K 9/00, A61K 31/58, A61K 45/06, A61Q 7/00, A61K 8/49, A61K 8/63, A61K 8/85, A61K 31/506, A61K 31/56, A61K 47/34, A61L 27/54, A61L 27/58

(54) **IMPLANTAT ZUM SUBKUTANEN IMPLANTIEREN**

(30) Priorität: 31.10.2018 DE 102018127200
(71) Anmelder: Kasperk, Christian, 69123 Heidelberg (DE); Haas, Andreas, 69207 Sandhausen (DE)
(72) Erfinder: Haas, Andreas, 69207 Sandhausen (DE)
(74) Vertreter: Wunderlich & Heim Patentanwälte Partnerschaftsgesellschaft mbB

(57) **Zusammenfassung**

Die Erfindung betrifft ein Implantat zum subkutanen Implantieren mit einem im Wesentlichen zylinder-, kegel- oder spiralförmigen Grundkörper, der eine Mantelfläche aufweist. Der Grundkörper besteht aus einem vollständig resorbierbar bioabbaubaren Polymer. Ferner ist das Implantat zum stumpfen Implantieren ausgebildet ist. Außerdem ist das bioabbaubare Polymer mit einem medizinisch relevanten Wirkstoff angereichert.

## Beschreibung

Die Erfindung betrifft ein Implantat zum subkutanen Implantieren eines Medikamententrägers und insbesondere ein Implantat zur Verwendung zur Behandlung von Erkrankungen, welch eine kontinuierliche Therapie benötigen.

Beispiele hierfür sind die Behandlung von Östrogenmangel-assoziierten Syndromen bei postmenopausalen Frauen, gynäkologisch oder endokrinologisch indizierten Daueröstrogenbehandlungen von Frauen jedweden Alters, beispielsweise auch Mann-zu-Frau-Transsexualität oder auch zur Behandlung des androgenetischen Haarausfalls, vor allem bei Männern. Da die Daueröstrogenbehandlung bei Frauen etablierte Routine in der endokrinologischen und gynäkologischen Praxis ist, wird im Folgenden exemplarisch nur detaillierter auf die Behandlung und Hintergründe einer pharmakologischen Dauerbehandlung der androgenetischen Alopezie mit einem Medikamenten-Träger-Implantat mit neuen technischen Eigenschaften eingegangen.

Die androgenetische Alopezie ist ein genetisch prädisponiertes Ereignis im Erwachsenenalter, insbesondere bei Männern. Sie führt zu einem irreversiblen Haarverlust des Kopf- beziehungsweise Haupthaares. Die Ursache hierfür ist ein Verkümmern der Haarwurzel und ein Ausbilden von Vellushaarfollikeln. Erklärt wird dies durch eine Veränderung der Dynamik im Haarzyklus, welche dann zur Vellus-Transformation des Haarfollikels führt.

Der Haarwachstumszyklus, welcher auch als Haarzyklus bezeichnet wird, teilt sich im Wesentlichen in drei Phasen, eine Anagenphase, eine Katagenphase und eine Telogenphase. Die Anagenphase ist eine aktive Wachstumsphase, die zwischen zwei Jahren bis zu etwa sechs Jahren dauern kann. Hier bildet sich ein neuer Haarfollikel der das Generieren eines Haares veranlasst. In dieser Phase wächst das Haar entsprechend über Jahre hinweg und nimmt an Länge zu.

Anschließend folgt ein kurzes Stadium, welches als Katagenphase bezeichnet wird und ein bis zwei Wochen andauert. Hier verkürzt sich der Haarfollikel und stößt das Haar ab.

Anschließend folgt die Ruhephase, die als Telogenphase bezeichnet wird, welche fünf bis sechs Wochen bis zu etwa 100 Tagen andauert. In dieser Phase regeneriert sich der Haarfollikel, sodass sich die nächste Anagenphase und somit die Bildung eines neuen Haares anschließen kann.

Bei der androgenetischen Alopezie tritt eine Verschiebung des Gleichgewichtes der verschiedenen Phasen untereinander auf. So nimmt die Dauer der Anagenphase allmählich ab während die Dauer der Telogenphase zunimmt. Da die Dauer der Anagenphase die Haarlänge bestimmt, wird die maximale Länge des neuen anagenen Haares kürzer als die seines Vorgängers. Dies führt schließlich nach mehreren Zyklen zu einer Miniaturisierung bis hin zur Verkümmerung der Haarfollikel und zu einem kahlen Aussehen (Glatzenbildung).

Man spricht hierbei mittlerweile den Papillen der Haut bei der Aufrechterhaltung des Haarwachstums eine fundamentale Rolle zu. Die Kommunikation zwischen den Papillen der Haut und den Haarfollikelzellen, die sich unter dem Einfluss von Androgenen, wie dem Hormon Testosteron, entfaltet, resultiert aus der Sekretion von Cytokinen und Wachstumsfaktoren aus den Papillen. Dabei haben diese Faktoren eine autokrine Wirkung auf die Hautpapillen selbst und eine parakrine Wirkung auf die Haarfollikelepithelzellen. Testosteron als ein Steroidhormon kann frei in die Zellmembran eindringen und wird im Zytoplasma durch die 5-α-Reduktase, hauptsächlich des Types II, in Dihydrotestosteron (DHT) umgewandelt. Das DHT bindet sehr viel stärker an den Androgenrezeptor (AR) als Testosteron und dieser Komplex wird, unterstützt durch die AR-Co-Aktivatoren, in den Zellkern transportiert. Dies führt im Zellkern zur Zielgentranskription und anschließend zur Translation der oben genannten Faktoren, die dann biologische Aktivität auf die Papille der Haut sowie auf den Haarfollikel ausüben und den Haarfollikelzyklus beeinflussen.

Zu diesen Faktoren zählen unter anderem der Insulin like growth factor (IGF-1), der basic fibroblast factor (bFGF), der vascular endothelial growth factor (VEGF), der Cytokine wie transforming growth factor beta 1 (TGFβ 1), der interleukin 1 alpha (IL - 1α) sowie der tumor necrosis factor alpha (TNF a). Viele Studien haben die Rolle von Androgenen sowie die parakrinen Interaktionen zwischen den Papillen der Haut und den Haarfollikeln als entscheidenden Vorgang bei der Miniaturisierung und Verkümmerung von Haarfollikeln beschrieben. Je höher die Konzentration von Androgenen und Androgenrezeptoren ist, desto mehr wirkt sich dies auf die Expression von Genen aus, die den Haarfollikelzyklus stören. Dies führt zu einem vorzeitigen Abbruch der Anagenphase, was mit einem vorzeitigen Eintritt in die Katagenphase verbunden ist. Die Katagenphase tritt als eine Konsequenz der verringerten Expression von den die Anagenphase erhaltenden Faktoren, wie den Wachstumsfaktoren IGF-1, bFGF und VEGF, auf. Zudem fördert eine erhöhte Expression von Zytokinen (TGFβ1, IL-1α und TNFa) die Apoptose, also den Zelluntergang des Haarfollikels.

Kürzlich wurde berichtet, dass das Peptid DKK-1 durch das Androgen DHT hochreguliert wird, was zur Hemmung der äußeren Wurzelscheidezellen des Haarfollikels und schließlich zur Induktion der Apoptose dieser Zellen führt.

Ein Therapieansatz besteht in der Hemmung der Umwandlung des natürlichen Androgens Testosteron in das lokal am Androgenrezeptor wirkstärkere Dihydrotestosteron (DHT) durch die Blockade des Enzyms 5-α-Reduktase, sodass die Expression der den Haarfollikelzyklus negativ beeinflussenden Faktoren gehemmt wird. Bekannt sind hier die Wirkstoffe Finasterid und Dutasterid.

Studien zur Anwendung von 1 mg Finasterid pro Tag oder auch zur Anwendung von 2 mg bis 10 mg Minoxidil pro Tag, zeigen eine hemmende Wirkung dieser Substanzen auf die Entwicklung der Alopezie, sofern eine tägliche und lebenslange Einnahme sichergestellt wird. Diese Studien zeigen einen Erfolg im Sinne einer Beendigung des Haarverlustes und zum Teil ein erneutes Wachstum von Haaren.

Auch die Substanz Dutasterid ist ein kompetitiver Hemmer der "Typ 1" und "Typ 2" 5-α-Reduktase und zeigt ebenfalls günstige Wirkungen auf die Verhinderung der androgenetischen Alopezie bei täglicher Einnahme in der Dosierung 0,5 mg.

Beruhigenderweise ist die Langzeitanwendung von 5-α-Reduktase Hemmern nicht mit einer nachteiligen Wirkung auf den Muskelstoffwechsel verbunden, da die anabole Wirkung von Testosteron am Muskel unabhängig von der Anwesenheit von Dihydrotestosteron ist. Auch immer wieder befürchtete sexuelle Nebenwirkungen bei der Langzeiteinnahme von 5-α-Reduktase Hemmern sind eher ungewöhnlich und haben eine allenfalls milde klinische Symptomatik und sind nach Absetzen der 5-α-Reduktase Hemmer, nach aktueller Studienlage, reversibel.

Auch die topische Anwendung des bekannten Antihypertensivums Minoxidil, einem Blutdrucksenker, führt als Nebenwirkung zu einem vermehrten Haarwachstum. Der Wirkmechanismus ist hierbei noch nicht endgültig geklärt. Vermutet wird, dass Minoxidil aufgrund der Erweiterung der Kapillaren die Durchblutung lokal am Haarfollikel fördert. Weiter wird vermutet, dass die Telogenphase des Haarzyklus verkürzt wird und hierdurch die Wachstumsphase (Anagenphase) schneller beginnt.

Wesentliche Voraussetzung für den Erfolg dieser vorhandenen Therapien ist aber die äußerst regelmäßige, konsequent tägliche und lebenslängliche Einnahme dieser Substanzen. Alle bisher eingesetzten beschriebenen Substanzen zum Verhindern der androgenetischen Alopezie können nur wirksam eingesetzt werden, wenn sie einerseits frühzeitig, beim ersten Auftreten von vermehrtem Haarausfall im Rahmen der hormonellen Umstellung im jungen Erwachsenenalter, und andererseits auch konsequent lebenslang eingenommen werden. Frühzeitig heißt hierbei beim ersten Auftreten von vermehrtem Haarausfall im Rahmen der hormonellen Umstellung im jungen Erwachsenenalter. Allerdings ist die tägliche orale Einnahme oder die tägliche topische Anwendung belastend.

Problematisch bei all diesen Therapieformen ist daher, dass für einen möglichen Behandlungserfolg eine äußerste kontinuierliche, idealerweise tägliche, Einnahme beziehungsweise Verabreichung notwendig ist, anderenfalls kann der Therapieerfolg nicht sichergestellt werden. Ein weiteres Problem besteht darin, dass, insbesondere bei der topischen Anwendung, auch dritte Personen mit dem Wirkstoff in Berührung kommen können. Je nach verwendetem Wirkstoff besteht hierbei die Gefahr, dass insbesondere Frauen hiermit in Kontakt kommen können, wobei beispielsweise ein einfacher Hautkontakt bereits ausreichend ist. Bei Schwangeren bewirkt Finasterid embryonale Fehlbildungen und dergleichen. Auch Zyklusstörungen sind bekannt.

Ähnliche Probleme bestehen auch bei der Therapie von anderen Leiden mit unterschiedlichen Wirkstoffen. Dies betrifft insbesondere Therapien mit Wirkstoffen, die eine belastende tägliche Einnahme zum Erreichen eines gewünschten Wirkspiegels erfordern und damit eine außerordentliche Patientencomlience erfordern aber auch Therapien mit Wirkstoffen, die toxisch für Mitmenschen sein können, mit denen man in direkten Kontakt kommt und welche dann dermal vom Gegenüber oder Partner ungewollt aufgenommen werden könnten.

Der Erfindung liegt daher die **Aufgabe** zugrunde, ein Implantat zu schaffen, welches zur kontinuierlichen Verabreichung von Wirkstoffen geeignet ist, jedoch möglichst geringen Aufwand beim Implantieren verursacht.

Diese Aufgabe wird erfindungsgemäß durch ein Implantat zum subkutanen Implantieren mit den Merkmalen des Anspruchs 1 gelöst.

Vorteilhafte Ausführungsformen der Erfindung sind in den Unteransprüchen angegeben.

Gemäß der Erfindung weist ein Implantat zum subkutanen Implantieren einen im Wesentlichen zylinder-, kegel- oder spiralförmigen Grundkörper auf, welcher eine Mantelfläche aufweist, wobei der Grundkörper aus einem vollständig resorbierbaren bioabbaubaren Polymer besteht. Ferner ist es vorgesehen, dass das Implantat zum stumpfen Implantieren ausgebildet ist und an der Mantelfläche des Grundkörpers, bei einem zylinder- oder kegelförmigem Grundkörper, hervorstehende Flügel vorgesehen sind, welche einstückig mit dem Grundkörper ausgebildet sind. Diese sind aus ähnlichem oder demselben Material wie der Grundkörper. Ferner ist vorgesehen, dass das bioabbaubare Polymer mit mindestens einem medizinisch relevanten Wirkstoff angereichert beziehungsweise versetzt ist.

Auf die Flügel kann, muss aber nicht, bei einem spiralförmigen Grundkörper verzichtet werden. Diese Ausführung kann ähnlich einem Korkenzieher oder einer Feder sein. Der Querschnitt des Grundkörpers kann rund oder auch mehrkantig sein.

Ein Grundgedanke der Erfindung kann darin gesehen werden, ein Implantat zu schaffen, welches in einen Körper implantiert wird und dort belassen werden kann. Dieses Implantat ist ferner durch den Einsatz von bioabbaubaren Polymeren vollständig resorbierbar. Dies bedeutet, dass das Implantat nur einmal in den Körper, bevorzugt subkutan, implantiert werden muss und sich während der Wirkdauer zersetzt beziehungsweise abgebaut wird. So wird verhindert, dass ein weiterer Eingriff notwendig ist, um das Implantat wieder zu entfernen. Ferner ist entsprechend der Erfindung vorgesehen, dass das Implantat zum stumpfen Implantieren ausgebildet ist. Wesentlich ist auch, dass durch die Formgebung Herausrutschen verhindert zumindest aber erschwert ist.

Im Sinne der Erfindung ist unter stumpfen Implantieren zu verstehen, dass der Schritt der Implantatbettschaffung entfallen kann und/oder das Implantieren im Wesentlichen atraumatisch erfolgt. Dies bedeutet, dass beim Einbringen des Implantats in einen Körper das Gewebe des Körpers im Wesentlichen verdrängt wird und nicht aktiv ein Hohlraum zur Aufnahme des Implantates geschaffen werden muss.

Schlussendlich wurde entsprechend der Erfindung erkannt, dass man das bioabbaubare Polymer mit einem oder mehreren medizinisch relevanten Wirkstoffen, also einer pharmakologischen Substanz, anreichern kann. Durch den kontinuierlichen Abbau des Polymers findet ein kontinuierliches Ausschütten beziehungsweise Freisetzen des oder der Wirkstoffe statt, so dass dieser kontinuierlich über eine gewisse Zeitdauer verabreicht werden kann und seine beziehungsweise ihre systemischen Wirkungen entfalten kann.

Vorteilhaft ist es, wenn die Flügel umlaufend am Umfang der Mantelfläche angeordnet sind und in einem radialen Querschnitt des Implantates im Wesentlichen eine dreiecksähnliche Fläche aufweisen. Die Flügel können beispielsweise auch geschwungen ausgebildet sein. Die Flügel können auch spiralartig ausgebildet sein, wobei sie mindestens einen Schraubengang ausbilden. Eine andere Möglichkeit ist, den Grundkörper in Form einer Spirale selbst vorzusehen. Eine weitere Ausbildungsform entsprechend der Erfindung ist, die Flügel in Form von Widerhaken vorzusehen, die ein Herausziehen oder insbesondere, eigenständiges, Herausrutschen des Implantates verhindern.

Wesentlich an allen möglichen Flügelformen ist jeweils, dass sie ein stumpfes Einbringen des Implantates in einen Körper ermöglichen bzw. unterstützen, jedoch idealerweise ein Zurückziehen, Zurückdrücken und/oder Zurückrutschen verhindern. In einer Ausführungsform können sie auch ähnlich zu Widerhaken ausgebildet sein, welche auch ein Zurückziehen, Zurückdrücken und/oder Zurückrutschen verhindern.

In einer vorteilhaften Ausführungsform ist das Implantat selbst einziehend und/oder selbst schneidend bei einer Rotation um die Längsachse des Grundkörpers ausgebildet. Insofern kann das Implantat als Schraubenimplantat betrachtet werden, welches sich ähnlich einer selbstschneidenden Schraube, nämlich durch Rotation um die Längsachse des Grundkörpers selbst in den Körper hineinzieht. Dies hat wiederum den Vorteil, dass auf das aufwändige Verfahren der Implantatbettschaffung mit den entsprechenden Problemen bei der Hohlraumerweiterung, beispielsweise Blutungen, Schmerzen, Infekte, verzichtet werden kann.

Beispielsweise kann das erfindungsgemäße Implantat so subkutan an einer klinisch sicheren, wenig störenden und ausreichend voluminösen Stelle, etwa im Bauchbereich, implantiert werden. Hierzu kann eine Lokalanästhesie erforderlich sein. Das Implantieren erfolgt beispielsweise mit einer minimalen Stichinzision, durch die anschließend das Implantat in das zum Beispiel subkutane Gewebe selbst einziehend eingeschraubt wird. Wie beschrieben, fallen so die notwendigen Schritte der Implantatbettschaffung durch einen speziell ausgestalteten Hohlraumerweiterer weg.

Ferner kann das Einbringen des Implantats mit vorgesehenen Widerhaken auch nur einzudrücken, ähnlich dem Einbringen eines Dübels, lediglich in Richtung Körperinnerem erfolgen, wobei des gegen zurückrutschen gesichert ist.

Prinzipiell kann das Implantat eine beliebige Resorptionsrate aufweisen. Bevorzugt beläuft sich diese auf mindestens drei Monate. Im Sinne der Erfindung wird unter Resorptionsrate die Zeit verstanden, die ein Körper, in den das Implantat eingesetzt ist, benötigt, um das Implantat vollständig abzubauen d. h. zu resorbieren. Bevorzugt ist es, wenn dieser Zeitraum möglichst lang ist, beispielsweise zwei oder drei Jahre beträgt. Als Komponenten der bioabbaubaren Polymere können beispielsweise Polylaktide, Polyglycoide, Polylactid-co-glycolide, Polycaprolactane oder Oxalat-vernetzte Oligocaprolactane verwendet werden.

Das Freisetzten der vorhandenen Wirkstoffe erfolgt durch Polymererosion, wobei im Wesentlichen zwischen zwei Formen, welche auch gleichzeitig auftreten können, unterschieden wird. Zum einen die Oberflächenerosion, bei der das bioabbaubare Polymer von außen nach innen abgebaut wird, zum anderen die Bulkerosion, bei der im Wesentlichen alle Polymermoleküle gleichmäßig abgebaut werden.

Allgemeine bekannte Faktoren, die den Abbau beeinflussen sind der pH-Wert, die Partikelgröße und die Wasserdiffusion in das Material. Bioabbaubare Polymere werden in wässrigen Medien zu Monomeren abgebaut. Diese werden metabolisiert und über die Lunge in Form von Wasser und Kohlenstoffdioxid abgeatmet. Ein weiterer Teil, welcher meist relativ klein ist, kann auch über den Urin ausgeschieden werden.

Wird das gewählte bioabbaubare Polymer aus mehreren verschiedenen Polymeren zusammengesetzt, beispielsweise Polylactid und Polyglycoid, so kann durch das Verhältnis der beiden Materialien zueinander die Abbaurate beeinflusst werden. Eine Eigenschaft, die im Wesentlichen zur Variation der Abbaurate beiträgt, ist die Hydrophilie der eingesetzten Polymere, da somit die aufgenommene Wassermenge in das Implantatmaterial beeinflusst werden kann und so die Abbaurate beeinflusst wird. In ähnlicher Weise kann das bioabbaubare Polymer aus Polycaprolactan und Oxalatvernetztem Oligocaprolactan zusammengesetzt sein, wobei wiederum das Verhältnis zueinander die Abbaurate bestimmt.

Es hat sich als vorteilhaft herausgestellt, wenn das Implantat keine spitze sondern eine zumindest leicht abgerundete oder etwas stumpfe Spitze aufweist. Anders ausgedrückt ist die Spitze eine gewebeschonende abgerundete Spitze. Dies trägt dazu bei, dass das stumpfe Implantieren im Sinne der Erfindung ermöglicht wird. So werden auch Gewebeschäden beim Einbringen des Implantates verhindert beziehungsweise vermindert, da wie bereits beschrieben, das Gewebe im Wesentlichen nicht geschnitten, sondern lediglich verdrängt wird. Hierdurch entstehen somit noch weniger Schäden im Gewebe, was zu einer besseren Verträglichkeit führt.

Grundsätzlich können beliebige medizinisch wirksame Wirkstoffe in das Implantat eingebracht sein. Zur Therapie der androgenetischen Alopezie sind hierbei beispielsweise ein kompetetiver Hemmer der Typ 1 und/oder Typ 2 5-α-Reduktase, insbesondere Dutasterid, Finasterid und/oder die Substanz Minoxidil geeignet. Wie bereits einleitend beschrieben ist es bekannt, dass diese Wirkstoffe den Haarausfall verlangsamen, im besten Fall sogar stoppen können. Bei der Verwendung des erfindungsgemäßen Implantats wird durch die Möglichkeit der konstanten Abgabe des Wirkstoffes über einen langen Zeitraum ein konstanter Wirkspiegel erreicht, so dass besonders gute Erfolge erzielt werden können.

Eine andere Möglichkeit ist, dass als medizinisch relevanten Wirkstoff Östrogene und/oder Androgenen verwendet wird. Östrogene kann beispielsweise zur Dauerbehandlung von postmenopausalen Östrogenmangelsyndromen oder auch zur Dauerbehandlung der Mann-zu-Frau-Transsexualität eingesetzt werden. Androgenen kann zur lebenslänglichen Dauerbehandlung der Frau-zu-Mann Transsexualität Verwendung finden.

Um eine konstante Abgabe des Wirkstoffes zu begünstigen, ist es vorteilhaft, wenn die bioabbaubaren Polymere derart gewählt sind, so dass ein konstanter Abbau über die gesamte Resorptionsrate realisiert wird. Hierzu bieten sich die bereits erwähnten Polymere wie Polylaktide und Polyglycoide oder Polycaprolactane und Oxalat-vernetzte Oligocaprolactane an.

Ferner betrifft die Erfindung ein erfindungsgemäßes Implantat zur Verwendung bei der pharmakologischen Dauerbehandlung und bevorzugt ein erfindungsgemäßes Implantat zur Verwendung bei der Behandlung von androgenetischem Haarausfall, von postmenopausalen Östrogenmangelsyndromen, der Mann-zu-Frau-Transsexualität und/oder der Frau-zu-Mann Trans-sexualität.

Zusammengefasst ist die Erfindung auch für Dauerbehandlung mit jedwedem anderen Wirkstoff, der lokal gewebsverträglich dauerhaft topisch freigesetzt werden kann geeignet. Vorteilhaft ist die Erfindung daher in Situationen, die eine häufig lebenslange pharmakologische Dauerbehandlung erfordern.

Wird beispielsweise das erfindungsgemäße Implantat mit Finasterid und/oder Dutasterid als Wirkstoff verwendet, wird das Enzym 5-α-Reduktase kontinuierlich blockiert, sodass es nicht mehr zum Entstehen schädigender Spiegel des den Haarfollikel terminal ausdifferenzierenden Dihydrotestosterons kommen kann. Eine nicht konsequent regelmäßige tägliche Einnahme des Finasterids oder Dutasterids, wie es bei der belastenden täglichen oralen oder topischen Anwendung der Fall ist, würde bedeuten, dass immer ein phasenweiser Anstieg der lokalen Dihydrotestosteron(DHT)-Konzentrationen mit den negativen Auswirkungen auf den sich terminal ausdifferenzierenden Haarfollikel auftritt. Dies hätte zur Folge, dass das Apoptose-Programm der Haarfollikelzellen und dadurch ein Verkümmern des Haarbalges sowie der Ausbildung der Alopezie Vorschub geleistet wird. Somit kommt es durch das Verwenden des erfindungsgemäßen Implantats nicht nur zur Verbesserung der Compliance, sondern auch zu einem Optimieren der Therapieeffizienz an sich, durch das Sicherstellen konstanter Wirkspiegel der relevanten Wirkstoffe.

Des Weiteren ist ein weiteres Problem der herkömmlichen Therapie gelöst, da die Kontaktmöglichkeit der im Implantat eingebrachten Substanzen zu Personen im Umfeld des Implantatträgers ausgeschlossen wird.

Mit dem erfindungsgemäßen Implantat beziehungsweise dessen Verwendung wird damit eine kontinuierliches und sicheres Verabreichen von Wirkstoffen ermöglicht, ohne den Aufwand beim Implantieren unnötig zu erhöhen und eine bessere Akzeptanz erreicht.

## Patentansprüche

1. Implantat zum subkutanen Implantieren
mit einem im Wesentlichen zylinder-, kegel- oder spiralförmigen Grundkörper, welcher eine Mantelfläche aufweist,
wobei der Grundkörper aus einem vollständig resorbierbar bioabbaubaren Polymer besteht,
wobei das Implantat zum stumpfen Implantieren ausgebildet ist,
wobei an der Mantelfläche des Grundkörpers, bei einem zylinder- oder kegelförmigem Grundkörper, hervorstehende Flügel vorgesehen sind, welche einstückig mit dem Grundkörper ausgebildet sind,
wobei das bioabbaubare Polymer mit einem medizinisch relevanten Wirkstoff angereichert ist.

2. Implantat nach Anspruch 1,
**dadurch gekennzeichnet,**
**dass** die Flügel umlaufend am Umfang der Mantelfläche angeordnet sind,
**dass** die Flügel in einem radialen Querschnitt des Implantates eine im Wesentlichen dreiecksähnliche Fläche aufweisen.

3. Implantat nach einem der Ansprüche 1 oder 2,
**dadurch gekennzeichnet,**
**dass** die Flügel spiralartig ausgebildet sind, wobei sie mindestens einen Schraubengang ausbilden.

4. Implantat nach einem der Ansprüche 1 bis 3,
**dadurch gekennzeichnet,**
**dass** das Implantat selbst einziehend und/oder selbst schneidend bei einer Rotation um die Längsachse des Grundkörpers ausgebildet ist.

5. Implantat nach einem der Ansprüche 1 bis 4,
**dadurch gekennzeichnet,**
**dass** es eine Resorptionsrate von mindestens drei Monaten aufweist.

6. Implantat nach einem der Ansprüche 1 bis 5,
**dadurch gekennzeichnet,**
**dass** es eine stumpfe oder eine abgerundete Spitze aufweist.

7. Implantat nach einem der Ansprüche 1 bis 6,
**dadurch gekennzeichnet,**
**dass** der medizinisch relevanten Wirkstoff ein kompetitiver Hemmer der Typ1 und/oder Typ2 5-α-Reduktase, insbesondere Dutasterid, Finasterid und/oder Minoxidil ist.

8. Implantat nach einem der Ansprüche 1 bis 6,
**dadurch gekennzeichnet,**
**dass** der medizinisch relevanten Wirkstoff Östrogene und/oder Androgenen ist.

9. Implantat nach einem der Ansprüche 1 bis 8,
**dadurch gekennzeichnet,**
**dass** die bioabbaubaren Polymere einen konstanten Abbau über die Zeit aufweisen.

10. Implantat nach einem der Ansprüche 1 bis 9,
**dadurch gekennzeichnet,**
**dass** als bioabbaubare Polymere Polylacitid und Polyglycoid oder Polycaprolactan und Oxalat-vernetzte Oligocaprolactone eingesetzt ist, wobei durch das Verhältnis der beiden Polymere zueinander die Resorptionszeit einstellbar ist.

11. Implantat nach einem der Ansprüche 1 bis 10 zur Verwendung bei der pharmakologischen Dauerbehandlung.

12. Implantat nach Anspruch 11 zur Verwendung bei der Behandlung von androgenetischem Haarausfall, von postmenopausalen Östrogenmangelsyndromen, der Mann-zu-Frau-Transsexualität und/oder der Frau-zu-Mann Trans-sexualität.
